# EUROPEAN PATENT APPLICATION

(11) **EP 1 547 582 A1**
(43) Date of publication of application: **29.06.2005**
(21) Application number: 03029813.7
(22) Date of filing: 23.12.2003
(51) Int. Cl.: A61K 9/127

(54) **Method of producing lipid complexed camptothecin-carboxylate**

(71) Applicant: MediGene Oncology GmbH, 82152 Planegg/Martinsried (DE)
(72) Inventor: The designation of the inventor has not yet been filed
(74) Representative: Weiss, Wolfgang, Dipl.-Chem. Dr.

(57) **Abstract**

A method for producing a cationic lipid complex comprising a camptothecin drug with enhanced physical and chemical stability, cationic lipid complexes obtainable by this method and pharmaceutical compositions thereof are disclosed.

## Description

Camptothecin (CPT) is a quinoline-based alkaloid, which can be isolated from the Chinese tree Camptotheca acuminata (Wall and Wani 1996). It was first described and tested as an anti-cancer drug in the 60ies and 70ies. Anti-tumor activity was noted in animal models and in clinical studies. However, patients experienced severe side reactions such as neutropenia, thrombocytopenia, haemorrhagic cystitis (Wall and Wani 1996). The therapeutic effect of camptothecin in humans had been questioned (Moertel, Schutt et al. 1972; Muggia, Creaven et al. 1972). It continued to be of high interest as a potential candidate for the development of an anti-cancer drug, and it was found that it has a particular mode of action, wherein binding to the topoisomerase I-DNA complex induces DNA breaks and cell death (topoisomerase I inhibitor) (Hsiang and Liu 1988).
A fundamental molecular property of CPT is its pH dependent equilibrium between the lactone and the carboxylate form. The lactone form is lipophilic, while the carboxylate, which predominates at physiological pH and above, is water-soluble. Since the lactone form is too lipophilic to be administered in an aqueous solution, initially, CPT was transformed into its water-soluble CPT carboxylate sodium salt (NCS 100880). However, due to severe side reactions and poor efficacy in preclinical/clinical studies, the development of the CPT carboxylate was not further pursued (Moertel, Schutt et al. 1972; Muggia, Creaven et al. 1972).

Due to these unfavourable properties of CPT-carboxylate, further efforts for the development of CPT based drugs were concentrated on the control of the equilibrium between the lactone and the carboxylate form. Current activities favour the development of CPT drugs with objectives to stabilize the lactone form and to find ways to administer it without difficulties (7).

Various strategies have been followed to stabilize the lactone ring and to concomitantly improve the solubility properties of the molecule in order to provide easier administration. Particularly, functionalisation of the original molecule to different types of derivatives, synthesis of prodrugs, and several types of administration have been pursued (Kehrer, Soepenberg et al. 2001). However, none of them has brought the desired results of resolving the above-described inherent difficulties of camptothecin as an anti-cancer drug.
In another approach, liposomes were used to protect CPT-lactone from converting into the carboxylate form. This was realized by encapsulation of CPT in a liposome under acidic conditions, or by embedding CPT into the lipid bilayer of a liposome in order to protect the lactone form from hydrolysis and from blood and serum interactions. In fact, by embedding CPT-lactone in the hydrophobic region of the vesicular lipid bilayer (US 5552156) the lactone form was not exposed to the aqueous environment and hydrolysis was significantly slowed down. However, only very low drug/lipid ratios could be achieved and therefore the necessary dosages for clinical use could not be realized.
In a further liposome-based approach, CPT-lactone was embedded into the lipid bilayer of a liposome comprising phospholipids, which contain unsaturated fatty acids (US 5834012). Thereby a stabilization effect was reported. It was proposed that the latter was due to the interaction of CPT in the lactone form with the unsaturated fatty acid chains of the lipids.

For pharmaceutical application a liposomal camptothecin preparation needs to contain a sufficient amount of camptothecin, since otherwise administration of a dose needed for pharmaceutical efficacy cannot be realized. Further, the preparation must be chemically and physically stable for a sufficient time in order to permit clinical application. In this context, several, eventually opposing, criteria must be fulfilled:
- it must be assured, that camptothecin is in an appropriate molecular state (CPT-lactone versus CPT-carboxylate),
- chemical degradation of camptothecin must be avoided,
- chemical degradation of other compounds of the preparation must be avoided; in case of lipid based preparations, a typical problem is lipid hydrolysis, which occurs favourably at high or low pH,
- physical stability must be ensured; colloidal stability must be provided and size distribution must be controlled,
- the preparation must be pharmaceutically active,
- in case of pharmaceutical preparations for iv injection, a certain particle number must not be exceeded.

Up to now however, no satisfying method for producing a physically and chemically stable liposomal camptothecin drug formulation has been reported. Especially due to the complexity of formulating camptothecin no manufacturing process with respect to the problem of upscaling and other aspects related to regular production of a pharmaceutical preparation has been provided.

Thus, the problem underlying the present invention was to provide an improved method for the production of a cationic liposomal preparation comprising a camptothecin drug. The cationic liposomal preparation should thereby have by a sufficient shelf life and in-use stability.

The solution to the above problem is achieved according to the invention by providing the embodiments characterized in the claims. The invention relates to a method of producing a cationic liposomal preparation comprising a camptothecin drug in its carboxylate form, comprising the steps of
a) providing cationic liposomes in an aqueous medium comprising the components
   i) at least one cationic lipid and optionally at least one amphiphile,
   ii) a camptothecin drug in its carboxylate form and
   iii) a cryoprotectant,
b) optionally homogenizing the liposomes of step a) at least once,
c) optionally sterile filtrating the liposomes of step a) or b),
d) dehydrating the liposomes of step a) b) or c) and
e) reconstituting the dehydrated liposomes of step d) in an aqueous medium,
   wherein said aqueous medium of step a) and/or of step e) comprises a pH active agent in a concentration of 0 mM to about 10 mM and has a pH between about 5 and about 9, preferably between about 6 and about 8.

Type, concentration of the pH active agent and the resulting pH may be different in step a) and step d).

Any one of the steps a) to e) can preferably performed protected from light, most preferably protected form light with a wavelength below 400 nm.

In the context of the present invention "camptothecin drug" refers to camptothecin itself or a derivative thereof. A camptothecin derivative is obtained by any chemical derivatization of camptothecin (see structure). A non-limiting list of possible camptothecin drugs is given under: http://dtp.nci.nih.gov as from Aug. 19, 2002. In the sketch of the molecule, the most frequent derivatisation sites are outlined as R₁-R₅.

Structure of camptothecin drugs:

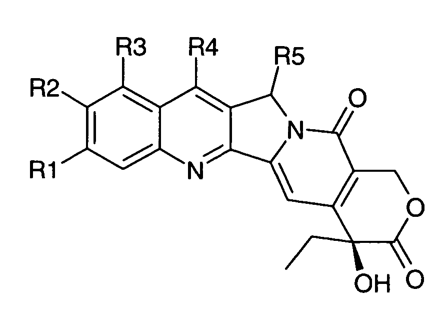

In table 1, typical examples for derivatization at different sites are listed. Camptothecin may be present as a hydrochloride. The lactone ring (E-ring) may be seven-membered instead of six-membered (homocamptothecins).

Derivatization can influence the properties of CPT to make the molecule more hydrophilic or more lipophilic, or that the lactone-carboxylate equilibrium is affected. In the context of the application of CPT as an anti-cancer drug, derivatization is intended to maintain or to increase activity.
For producing the inventive composition, camptothecin or any suitable camptothecin derivative in the carboxylate form is appropriate. Subsequently the inventive composition will be outlined with camptothecin as working example, however, the illustration also counts for any camptothecin derivative, according to its molecular properties.

The cationic liposomal preparation comprises a camptothecin drug in its carboxylate form, preferably camptothecin itself, but also 10-OH-CPT, SN-38 or other derivatives. The camptothecin drug is thereby present in an amount of about 0.1 mol% to less than about 100 mol% with respect to the amount of cationic lipid. In other embodiments it is present from about 1 mol% to about 50 mol%. In other embodiments, a camptothecin drug is present in about 3 mol% to about 30 mol% and in even other embodiments it is present in about 5 mol% to about 10 mol%.

Useful cationic lipids thereby include:
DDAB, dimethyldioctadecyl ammonium bromide; N-[1-(2,3-dioleoyloxy)propyl]-N,N,N-trimethyl ammonium (DOTAP); N-[1-(2,3-diacyloxy)propyl]-N,N,N-trimethyl ammonium, (including but not limited to: dioleoyl, dimyristoyl, dilauroyl, dipalmitoyl and distearoyl; also two different acyl chains can be linked to the glycerol backbone); N-[1-(2,3-dioloyloxy)propyl]-N,N-dimethyl amine (DODAP); N-[1-(2,3-diacyloxy)propyl]-N,N-dimethyl amine, (including but not limited to: dioleoyl, dimyristoyl, dilauroyl, dipalmitoyl and distearoyl; also two different acyl chains can be linked to the glycerol backbone); N-[1-(2,3-dioleyloxy)propyl]-N,N,N-trimethylammonium (DOTMA); N-[1-(2,3-dialkyloxy)propyl]-N,N,N-trimethyl ammonium, (including but not limited to: dioleyl, dimyristyl, dilauryl, dipalmityl and distearyl; also two different alkyl chains can be linked to the glycerol backbone); dioctadecylamidoglycylspermine (DOGS);, 3□-[N-(N',N'-dimethylaminoethane)carbamoyl]cholesterol (DC-Chol); 2,3-dioleoyloxy-N-(2-(sperminecarboxamido)-ethyl)-N,N-dimethyl-1-propanaminium trifluoro-acetate (DOSPA); β-alanyl cholesterol; cetyl trimethyl ammonium bromide (CTAB); diC14-amidine; N-*tert*-butyl-N'-tetradecyl-3-tetradecylaminopropionamidine; 14Dea2; N-(alpha-trimethylammonioacetyl)didodecyl-D-glutamate chloride (TMAG); O,O'-ditetradecanoyl-N-(trimethylammonioacetyl)diethanolamine chloride; 1,3-dioleoyloxy-2-(6-carboxy-spermyl)-propylamide (DOSPER); N,N,N',N'-tetramethyl-N,N'-bis(2-hydroxylethyl)-2,3-dioleoyloxy-1,4-butanediammonium iodide; 1-[2-(acyloxy)ethyl]2-alkyl(alkenyl)-3-(2-hydroxyethyl)-imidazolinium chloride derivatives as described by Solodin et al. (1995) Biochem. 43:13537-13544, such as 1-[2-(9(Z)-octadecenoyloxy)ethyl]-2-(8(Z)-heptadecenyl-3-(2-hydroxyethyl)imidazolinium chloride (DOTIM), 1-[2-(hexadecanoyloxy)ethyl]-2-pentadecyl-3-(2-hydroxyethyl)imidazolinium chloride (DPTIM), 2,3-dialkyloxypropyl quaternary ammonium compound derivatives, contain a hydroxyalkyl moiety on the quaternary amine, as described e.g. by Felgner et al. (Felgner, Kumar et al. 1994) such as: 1,2-dioleoyl-3-dimethyl-hydroxyethyl ammonium bromide (DORI), 1,2-dioleyloxypropyl-3-dimethyl-hydroxyethyl ammonium bromide (DORIE), 1,2-dioleyloxypropyl-3-dimetyl-hydroxypropyl ammonium bromide (DORIE-HP), 1,2-dioleyloxypropyl-3-dimethyl-hydroxybutyl ammonium bromide (DORIE-HB), 1,2-dioleyloxypropyl-3-dimethyl-hydroxypentyl ammonium bromide (DORIE-Hpe), 1 ,2-dimyristyloxypropyl-3-dimethyl-hydroxylethyl ammonium bromide (DMRIE), 1,2-dipalmityloxypropyl-3-dimethyl-hydroxyethyl ammonium bromide (DPRIE), 1,2-disteryloxypropyl-3-dimethyl-hydroxyethyl ammonium bromide (DSRIE); cationic esters of acyl carnitines as reported by Santaniello et al. [US5498633].
In a preferred embodiment the cationic lipid is selected from a quaternary ammonium salt such as N-[1-(2,3-diacyloxy)propyl]-N,N,N-trimethyl ammonium, wherein a pharmaceutically acceptable counter anion of the quaternary amino compound is selected from the group consisting of chloride, bromide, fluoride, iodide, nitrate, sulfate, methyl sulfate, phosphate, acetate, benzoate, citrate, glutamate or lactate.

Preferred liposomes of the present invention comprise DOTAP, DODAP, analogues of DOTAP or DODAP or any other cationic lipid. Cationic liposomes of the present invention comprise at least an amount of about 30 mol% cationic lipids, preferably about 40 mol%, more preferably about 50 mol%, even more preferred about 60 mol%, about 70 mol%, about 80 mol%, or about up to 99.9 mol% and are characterized by having a positive zeta potential in about 0.05 M KCI solution at about pH 7.5 at room temperature.

Amphiphiles used in the present invention are selected from sterols such as cholesterol or lipids such as phospholipids, lysolipids, lysophospholipids, sphingolipids or pegylated lipids with a neutral net change. Useful neutral lipids thereby include: Phosphatidic acid, phosphatidylserine, phosphatidylglycerol, phosphatidylinositol (not limited to a specific sugar), fatty acids, sterols containing a carboxylic acid group, cholesterol, 1,2-diacyl-sn-glycero-3-phosphoethanolamine, including but not limited to dioleoyl (DOPE), 1,2-diacyl-glycero-3-phosphocholin and sphingomyelin. The fatty acids linked to the glycerol backbone are not limited to a specific length or number of double bonds. Phospholipids may also have two different fatty acids. In a preferred embodiment the neutral amphiphile is diacylphosphatidylcholine.

Amphiphiles are present in the inventive liposomes in an amount of up to about 70 mol%, preferably, about 60 mol%, more preferably about 50 mol%, even more preferred about 40 mol%, about 30 mol%, about 20 mol%, or 10 mol% or less.

A suitable aqueous solution according to step a) of the present invention comprises water, optionally a pH active agent and a cryoprotectant and has a pH value between about 5 and about 9, preferably between about 6 and about 8. Suitable pH active agents may be composed of inorganic or organic ions or combination thereof. In case of inorganic the cationic part may comprise for example sodium, potassium, ammonium or hydronium ions and the anionic part may comprise carbonate, phosphate or sulphate or hydroxy ions. The typical example is sodium hydrogen carbonate. Other pH active agents can be selected from bases, which are used for buffer systems, such as Tris, Bis, HEPES or amino acids. The respective buffer systems might be adjusted to a certain pH with acids or bases such as HCl or NaOH.

The pH active compound is present at a concentration, at which no or only very low buffer capacity is present. The concentration of the pH active agent is of 0 mM to about 10 mM, preferably of less than about 5 mM, more preferably of less than about 1 mM, even more preferably of less than about 0.5 mM and most preferably of about 0.25 mM.

The cryoprotectant is selected from a sugar or an alcohol or a combination thereof such as trehalose, maltose, sucrose, glucose, lactose, dextran, mannitol or sorbitol and used in the range of up to about 20 % (m/v). Preferably the stabilizing agent is used in the range of about 0.1 % (m/v) to about 20 % (m/v) and most preferably in the range of about 5 % (m/v) to about 15 % (m/v) with respect of the total volume of the liposomal dispersion further in step b).

Preparing a cationic liposomal preparation according to step a) of the inventive method comprises several methods well known in the art. In a preferred embodiment of the present invention organic solvent injection, or a mechanical dispersion method such as high-pressure homogenization, extrusion or homogenization or stirring is performed.

According to the organic solvent injection method cationic lipids and optionally amphiphiles are dissolved in an organic solvent or a mixture of different organic solvents which are selected from alcohols (such as ethanol or *tert*-butanol), ether or other suitable water miscible or volatile organic solvents. The organic solvent injection is performed by dissolving the cationic lipids and optionally amphiphiles in a water miscible volatile solvent, preferably ethanol, and injecting this solution into an aqueous solution comprising the camptothecin drug in its carboxylate form and a cryoprotectant. The organic phase should thereby not exceed about 5 % (m/v) in the final liquid liposomal preparation before freeze-drying.

Another possibility for preparing liposomes in an aqueous solution is performed by mechanical mixing of the components. This can be achieved by any method of mechanical homogenization. A number of instruments for such processing at laboratory and at industrial scale is known in the art. Examples are (micro-) homogenizers, high-pressure homogenizers, extruders, compounders or suitable stirrers.
The dispersion process can be performed in the way, that all components, including the aqueous solution, are homogenized in a single step, or alternatively that the lipophilic components are firstly homogenized and secondly dispersed in a solution of the water-soluble components.

Adjusting the size of liposomes is often performed by sonication in the art. However, in the inventive method homogenising in step b) is preferably performed by extrusion, filtration through membrane filters and/or high speed homogenization and mostly preferred by extrusion through a membrane with a pore size of about 200 nm under pressure. Membranes with other pore sizes such as 50 nm, 100 nm, 150 nm, 400 nm or any other pore size well known in the art may be used. Filtration through membrane filters maybe performed by filtration through membranes composed of polycarbonate (PC), PVDF, PES, Nylon-filters but also other materials may be used if defined to be suitable. Different materials and different pore sizes maybe combined in a way to obtain a solution which maybe processed by a sterilizing grade filtration.

For pharmaceutical use, it is a prerequisite that the liposomal preparation can be sterilised through a sterilizing grade filter. Methods for sterilizing liposomes should be destructive for micro-organisms, but should not affect physicochemical characteristics of the liposomal preparation in an unfavourable manner. Sterilising pharmaceutical products in the art is performed by autoclaving, e.g. at 134 °C for a minimum of 5 min or at 121 °C for a minimum of 15 min. Under these harsh conditions liposomes often show degradation at considerable content, e.g. as agglomeration of liposomes, change of liposomal size or size distribution, hydrolysis/oxidation of lipids, chemical degradation or undesired release of the lipophilic compound from the liposomes. Therefore, sterile filtration and aseptic filling are preferred methods to obtain a pharmaceutical liposomal product for parenteral application. Typically, sterilizing grade filtration is performed once or repeatedly through a membrane with a pore sizes in the range of 100 to 450 nm. Materials commonly used are cellulose derivatives such as cellulose acetate or polyvinyl membranes like PVDF, PES or Nylon but also other materials may be used if defined to be suitable.

Ultrafiltration processes may also be used to remove undesired compounds from the liposomal preparation, such as reagents or solvents used in the manufacturing process, or not liposomally loaded lipophilic compound. The pore size of the filter is preferably between the liposomal diameter (typically > 60 nm) and the compound to be removed (typically < 5 nm). Depending on the size difference ultra filtration (1 - 1000 kDa molecular weight cut-off) or micro filtration (0.02 - 1 µm) may be used. Instead of a dead end filtration more convenient techniques have been developed like dialysis or cross flow filtration.

After step c), dehydration (step d) is performed. The preparation is dehydrated and reconstituted prior to use with an aqueous solution such as pure water or a solution comprising a pH active agent.

During reconstitution, dried liposomes are resuspend with water optionally comprising a pH active agent of less than about 10 mM, preferably of less than about 5 mM, more preferably of of less than about 1 mM, even more preferably of less than about 0.5 mM and most preferably of about 0.25 mM while the physicochemical stability of the camptothecin drug in the liposomal membrane is not jeopardized. Reconstitution behaviour may be examined e.g. by visual assessment, microscopy or light blockage measurements.

The inventive method allows the production of cationic liposomes having a positive zeta potential in about 0.05 M KCl solution at about pH 7.5 at room temperature, preferably in the range of about 25 mV to 100 mV, more preferably in the range of about 30 mV to 70 mV and even more preferably in the range of about 35 mV to 65 mV.

The polydispersity index (size distribution coefficient, PI-value) of the inventive cationic liposomal preparation are below about 0.6, preferably below about 0.5, more preferred below about 0.4 and most preferred below about 0.3.

Cationic liposomes prepared by the inventive method and the cationic liposomes disclosed in the present invention have a diameter in the range of about 50 to about 400 nm, preferably about 50 to about 350 nm and more preferably about 100 to about 300 nm.

It is a feature of the present invention that the camptothecin drug is stabilized in its carboxylate form within the liposome. The inventive method provides a cationic liposomal preparation
wherein the content of CPT in its lactone form is below about 10% (% means molar fraction of the total CPT content), preferably below about 8% and more preferably below about 6% and most preferably below about 4% with respect to total CPT.

The inventive method provides an improved manufacturing process for a cationic liposomal preparation comprising a camptothecin carboxylate drug with a physical and chemical stability of its constituent components during the time of manufacturing, storing and applying the preparation to a subject in need thereof, such is a shelf life of at least about one month and an in use stability of at least 2 hours.

Unless defined otherwise, all technical and scientific terms used in this specification shall have the same meaning a commonly understood by persons of ordinary skill in the art to which the present invention pertains.

"About" in the context of amount values refers to an average deviation of maximum +/- 30 %, preferably +/- 20 % based on the indicated value. For example, an amount of about 30 mol% cationic lipid refers to 30 mol% +/- 9 mol% and preferably 30 mol% +/- 6 mol% cationic lipid with respect to the total lipid/amphiphile molarity.

"Amphiphile" refers to a molecule consisting of a water-soluble (hydrophilic) and an organic solvent -soluble (lipophilic) moiety. A suitable amphiphile of the present invention can be cationic, neutral or anionic with regard to the net charge of the hydrophilic moiety (head group). A cationic amphiphile has a positive net charge, a neutral amphiphile a neutral and an anionic amphiphile an anionic net charge. An amphiphile, such as used in the present invention, is selected from sterols such as cholesterol, phytosterol or lanosterol or lipids such as lysophospholipids, sphingolipids or pegylated lipids such as 1,2-diacyl-sn-glycero-3-phosphoethanolamine, including but not limited to dioleoyl (DOPE), 1,2-diacyl-glycero-3-phosphocholines, sphingomyelin. Pegylated lipids refer to lipids bearing one ore more polyethylene glycol residues.

"Aqueous solution" refers to any solution comprising water and optionally at least one suitable additive, which is completely dissolved in water. Such additives may be buffers or their individual components, sugars, alcohols, stabilizing agents.

"Cationic lipid" refers to an amphiphile that has a positive charge (at physiological pH) as measurable by instrumentation utilized at the time of the measurement. Where there are fatty acids or alkyl chains present on the cationic lipid, they could be 12-24 carbons in length, containing up to 6 unsaturations (double bonds), and linked to the backbone by either acyl or ether linkages; there could also only be one fatty acid or alkyl chain linked to the backbone. Where there is more than one fatty acid or alkyl chain linked to the backbone, the fatty acids could be different (asymmetric). Mixed formulations are also possible.

"Cationic liposomes" are liposomes which have a positive net charge. They can be prepared from one or more cationic lipids, or in admixture with one or more further amphiphiles. They can further comprise compounds which are embeded or encapsulated in the liposome.

"Cationic liposomal preparation or formulation" refers to either a dehydrated liposomal preparation or formulation or a liposomal dispersion. The terms "cationic liposomes", "cationic liposomal preparation", "cationic liposomal dispersion" or "cationic liposomal formulation" are used synonymously herein.

"Chemical stability" can be defined by HPLC/LC-MS/MS and typically means less than 5 % degradation products of the respective component.

"Compound loaded into the liposome" or "liposomally loaded compound" or liposomal compound" is used synonymously and refers to a compound that is either integrated in the lipid bilayer of the liposome or associated with the lipid bilayer of the liposome of the liposomal preparation.

"Concentration" of x mol% of an amphiphilic or lipophilic compound refers to the mol fraction of this compound of the total lipid concentration. Concentrations of water-soluble compounds are given in % (m/m) or % (m/v) of the total preparation.

"Liposomal dispersion" refers to liposomes within an aqueous solution. The terms "liposomal suspension", "liposomal preparation" or "liposomes" may be used synonymously.

"Liposomes" refer to microscopic spherical membrane-enclosed vesicles (50-2000 nm diameter), which consist of one or several lipid bilayers as central structural unit. Liposomes are also referred to as lipid vesicles. Liposome forming molecules are lipids or amphiphiles, which comprise a hydrophobic and a hydrophilic moiety in a suitable relation.

"pH active agent" refers to a compound which, after adding it to an aqueous solution, can change the pH of the solution. Typical pH active compounds are acids, bases, or salts thereof (both inorganic or organic). Also buffers and compounds as amino acids are pH active agents in this context.

"Physicochemical stability" and "Physical stability" refers to the physicochemical and physical state of the respective compound. In case of nanoparticulate colloidal dispersions this refers for example to the particle size and the size distribution within a preparation over all. Other parameters are phase state and molecular aggregation within a preparation.

"PI value" refers to the Polydispersity Index which refers to the particle size distribution in a liposomal dispersion as measured by dynamic light scattering techniques, e.g. with a Malvern Zetasizer 1000 or 3000.

"Stabilizing agent" refers to an agent that inhibits chemical or physical degradation within the preparation.

"Total lipid concentration" refers to the concentration of the sum of lipids and amphiphiles.

"Zeta potential" refers to a surface potential of a particle such as a colloidal particle measured with an instrument such as a Zetasizer 3000 using Laser Doppler micro-electrophoresis under the conditions specified. The zeta potential describes the potential at the boundary between bulk solution and the region of hydrodynamic shear or diffuse layer.

The advantages of the present invention are as follows:
- The inventive preparation is pharmaceutically active
- minimization of lipid degradation (hydrolysis), camptothecin degradation, camptothecin lactone formation and particle formation after reconstitution of a lyophilized preparation,
- high physical and chemical stability of the constituents of the cationic liposomal preparations,
- less than about 6 % of the lactone form of the loaded camptothecin drug,
- manufacturing of loaded liposomes on production scale of at least about 60 liters is possible,
- long shelf life of at least about one month,
- high in use stability of at least 2 hours after reconstitution (in aqueous solution).

Stability of the camptothecin drug and the lipids and amphiphiles during steps a) to d) and reconstitution step e) of the present invention is controlled by any of the following means:
- controlled pH in the aqueous phase
- controlled (low) temperature
- controlled (high) speed of manufacturing and/or application.

The inventive method allows physical and chemical stabilization of all constituents while the liposome is in an aqueous environment. In this context it is of particular importance, that the components of the inventive preparation have contrary requirements for stability. For the amphiphile components, best stability is achieved in the pH range between 5 and 7. For camptothecin carboxylate in an aqueous solution, a pH value above 7 is necessary to prevent lactone formation. Therefore, it seems impossible to find conditions for a pharmaceutically suitable liposomal CPT-carboxylate preparation, wherein both components are most stable. Within a limited range of conditions, degradation may be slowed down sufficiently to enable administration if this occurs fast enough after production (eg some days or weeks). To this end the pH and other conditions must be fixed very accurately, and buffering with a sufficient buffering strength is necessary. This is obtained by adding buffers at sufficient concentration such as at least 20 mM, better 50 mM.
Lyophilization of the preparation could be a way to obtain longer shelf life. However, after lyophilising and reconstituting a buffered (stabilized) preparation, a very high number of particles in the size range > 10 µm and > 25 µm can be observed. Such preparation however, cannot be used for pharmaceutical iv applications.

Surprisingly, it was found that liquid preparations which had not been stabilized with a buffer and which are therefore in a state of very poor stability, can be lyophilised and reconstituted thereafter without critical particle formation in the liquid preparations. Lyophilisates obtained this way can be stored for several months, and after reconstitution no critical chemical or physical degradation is found. The characteristics of the liquid formulations which are obtained after reconstitution of these lyophilized preparations, are very similar to those of the formulations before lyophilization. This means, they are still metastable, that is stable for a limited time. The stability of some hours after reconstitution however is sufficient as an in-use stability for application to a patient.
Thus, only formulations which had not been stabilized with a buffer in the liquid state could be lyophilised, stored and reconstituted without any detrimental effects for the pharmaceutical preparation.

The in-use stability can be further improved by adding a pH active agent to the preparation after reconstitution or by direct reconstitution of the lyophilised preparation with a solution comprising the pH active agent at a concentration of below 10 mM.

With the inventive method the large-scale production of physicochemically stable cationic liposomes comprising a camptothecin carboxylate drug is disclosed for the first time.

Most physiochemical stability of the liposomal preparation can be achieved if at least one of the steps a) to e) of the inventive method is performed at a temperature of below about 15°C, preferably below about 10 °C and more preferably below about 8°C and under avoidance of light.

In a further preferred embodiment of the present invention a stabilizing agent such as an antioxidant can be present during at least one of the steps a) to e). Suitable stabilizing agents are selected from alpha-tocopherol or vitamin C.

Another object of the present invention is to provide a cationic liposomal preparation comprising a camptothecin drug in its carboxylate form and optionally a pH active agent of up to about 10 mM in an aqueous solution, wherein said solution has a pH value between about 5 and about 9, preferably between about 6 and about 8, as well as a cationic liposomal preparation obtainable by the inventive process. The cationic liposomal preparation of the present invention is suitable for the manufacturing of a pharmaceutical composition, which can be in can be in a dry, lyophilized form or in the form of a liquid suspension. The lyophilized form is preferred, because it can be stably stored for periods up to several months. Suspensions of the pharmaceutical composition of the present invention in low acidic pH (buffered or acidified) are stable for several hours, depending upon the temperature, compound content, and phospholipid/amphiphile constituents.

Another object of the present invention is to provide a pharmaceutical composition comprising any one of the inventive liposomal preparations together with a pharmaceutically acceptable carrier, diluent and/or adjuvant.

The pharmaceutical composition of the present invention is active in the field of cancer treatment as well as several other acute or chronic diseases, and in general in the treatment of diseases associated with enhanced angiogenic activity by administering the composition to patients in an effective amount. The liposomes of the present invention may be administered alone or in combination with suitable pharmaceutical carriers or diluents. Suitable application forms are parenteral routes of administration such as intramuscular, intravenous, intraperitoneal as well as subcutaneous administration. Dosage forms suitable for parenteral administration include solutions, suspensions, dispersions, emulsions and the like well known in the art.

In light of the foregoing general discussion, the specific examples presented below are illustrative only and are not intended to limit the scope of the invention. Other generic and specific configurations will be apparent to those persons skilled in the art.

### Examples

### Example 1: Lab scale lyophilization of DOTAP/CPT

In this section, experiments concerning the lyophilizability of CPT/DOTAP preparations are summarized. For liquid preparations, it has been shown, that by buffering with 10-50 mM buffer (Tris/HCl) a stability of several weeks could be obtained. The pH of 7.5 was selected, because at higher pH lipid hydrolysis occurred and at lower pH the CPT transformed to a certain extend into the lactone form and precipitated. Higher buffer concentrations were favourable in order to keep the pH in the desired range.
Stabilities longer that some weeks were difficult to achieve because of partial lipid hydrolysis or CPT lactone formation.
In order to further extend the shelf life of CPT/DOTAP suspensions, lyophilization was tested. The aim was to find a way to lyophilize the CPT/lipid complex suspension at the buffer conditions with best stability.
Unfortunately, lyophilization of the buffered preparation yielded not the desired results. After reconstitution a very high number of particles > 10 µm and > 25 µm were present in the suspensions. With such high particle numbers, iv application of the preparation is not possible.

Unexpectedly, lyophilization was found to be possible without buffer, under conditions where the liquid suspensions were highly metastable with respect to lactone formation and precipitation, with a stability of only several hours or few days. After reconstitution of such lyophilisates, preparations with only low particle numbers > 10 µm and > 25 µm were found. The particle size distribution as measured by DLS was not significantly altered with respect to the state before lyophilization. Chemical composition after reconstitution was not affected. After reconstitution, preparations with an in-use stability of several hours were obtained, sufficient for the application to a patient. The lyophilisates were stored for several months without affecting the size distribution and the chemical composition.

In that way, DOTAP/CPT preparation with a lipid concentration of up to 30 mM and with a CPT concentration of up to 1.5 mM were lyophilized.
Subsequently, an exemplary list of experiments is documented.

### Preparation lipid stock solution

A 400 mM lipid stock solution in Ethanol was used. For 1 ml of the stock solution 279.42 mg of DOTAP was weighed in a graduated 1 ml flask and the flask was filled up to the mark with ethanol. After gentle mixing the solution was stored at 2-8 °C.

### Preparation CPT-carboxylate stock solution

Dry Camptothecin in the lactone form was weighted and put into a graduated flask and the flask was filled up to the mark with a NH₃: EtOH 1:4 solution.

After one hour the CPT-carboxylate had formed and dissolved in the solution. The volume with the needed mass of CPT for the preparation was added to a 500 ml round bottom flask. The solvent was evaporated carefully in a rotatory evaporator by applying a pressure of 250 mbar for about 15 minutes at 40°C. Then the pressure was lowered to 10 mbar for about 15 min. The dry film was reconstituted with a solution of 8-9% treahalose (m/v), depending on the experiment. The solution could further contain 10-50 mM tris (adjusted with HCl to pH 7.5) by gently shaking the flask.

### Preparation of the DOTAP/CPT preparations

### Ethanol injection

The CPT/ carboxylate solution was cooled with an ice bath and stirred with a magnetic stirrer. The 400 mM lipid stock solution was injected slowly using a 1 ml Hamilton syringe. The preparation was stirred for another 5 minutes in the ice bath.

### Extrusion

The preparation was extruded 5 times through a 220 nm PVPH membrane (Poretics, Polycarbonate, OSMONICS INC.) at 4 °C. Applied pressure was 5-6 bar.

### Lyophilization

Lyophilization was performed by a standard procedure as described in the art.

### Reconstitution

The samples were reconstituted with 1.96 ml pure water and agitated for a short time. After ten minutes they were shaken on the minishaker for 15 seconds and after another 20 minutes of waiting the reconstitution were finished. In some cases the samples were reconstituted with a 10 mM tris/HCl buffer solution, pH 7.5.

### Results

### Physicochemical characterization

### Liposomal size (DLS)

Most measured preparations had after extrusion and lyophilisation a liposomal size between 140 nm and 180 nm. Polydispersity indeces (PI) were between 0,10 and 0,27.
After lyophilisation polydispersity indeces increased to a value between 0,15 and 1,00.

### Non-visible particles

The liquid preparations after extrusion were clouded. But the same preparations after lyopilization were much cloudier particularly the preparations made with Tris-buffer (8,5 % trehalose with 10 mM tris) or reconstituted with tris-buffer.

In Tab. 2 particle numbers from preparations reconstituted with 0 mM, 10 mM and 50 mM in the liquid preparations before lyophilization and in the suspensions, which were obtained after reconstitution of the lyophilisates are shown.

The results show, that in the samples without buffer, the particle number are much lower that in case of more that 10 mM buffer present in the suspensions.

### Reconstitution of lyophilizates with Tris buffer

In Tab. 3 results from reconstituting lyophilisates from suspensions without buffer with water and with 10 mM Tris/HCl, pH 7.5 are given.

Reconstitution of buffer-free lyophilisates with 10 mM Tris/HCl, pH 7.5 induced particle formation.

### Lyophilized preparations

Production of the lyophilized liposomal preparations was performed as described earlier. In table 4 results of the PAMAS and DLS measurements can be found.

### Example 2: Manufacturing process (scale of 3I)

This example describes the manufacturing process on a 3I-scale. Identical parameters (including filter systems: pore size and surface size of the membrane) have been used to for producing 66l without any problems. All steps are performed with sterilized material.

### Liquid formulation

### Ethanolic DOTAP stock solution

36.325g DOTAP-CI wwas weighted in a sterile flask and was dissolved in 72.189g ethanol. Final DOTAP concentration is 400mM. The ethanolic solution is stored before use at 4°C. Stability (no DOTAP degradation) has been shown to be at least 1-2 months.

### CPT-Na stock solution

1.045g CPT lactone was weighted in a sterile round bottom flask (250ml). 20% of the needed volume water, to reach a final CPT concentration of 25mM, was added and 3.34g 1 N NaOH was added to the inhomogeneous mixture. The amount of NaOH is selected to reach a molar ratio of CPT/NaOH of 1:1.05.
The mixture is intensively stirred and heated to a temperature of 50°C. It takes about 1 hour until a homogeneous solution is formed, which means that CPT lactone has been quantitatively converted into its water-soluble carboxylate form.
Further studies has proofed that increasing the temperature up to 90°C accelerates the conversion into the CPT carboxylate form. Stirring for at least 4-6 hours has been proofed to be not critical for the chemical stability of CPT.
The solution is cooled down to room temperature and can be stored light-protected and at 4°C for at least 24hours. (Long time storage stability of the final CPT-Na stock solution is discussed later)
The pH of the final solution is between 10 and 11.5.

### CPT-Na-trehalose solution

99.65ml of the CPT-Na solution, 299.26g trahalose (as dihydrate) and 2908.82g water were stirred 1 h in a closed steel vessel. The solution is cooled down to 4°C. The used water has a temperature of 4°C.
The CPT-Na-trehalose solution is filtered through a 0.45µm PVDF membrane filter (Milipak60) to remove non-visible particles which could clog the extrusion membrane at a later manufacturing step.
The filtration process is performed in steel pressure vessels and at 4°C.

### Ethanol injection

112.5ml of the cooled (4°C) ethanolic DOTAP stock solution were injected with a injection rate of 250ml/min (into the cooled and filtered CPT-Na-trehalose solution. Injection is performed with a perfusor. The liposomal raw dispersion is stirred with a stirrer (395rpm) for about 1 h. The pH of the raw dispersion is measured. If the pH is below 6.5, this is usually the case, about 200µl 0.1N NaOH is added to increase the pH to 7.0-7.5. This in-process-control is crucial because earlier experiments clearly showed that manufacturing at lower pH than 6.5 leads to clogged filter membrane during later extrusion steps.

It is preferred that the ethanol injection and all further manufacturing steps are performed under strict avoidance of light and more preferably at a temperature below 15°C.

### Extrusion

Three extrusion steps were performed using a closed system of two steel vessels and filter unit containing 0.22µm polycarbonate membranes (Memtrex, Osmonics). All extrusion steps are performed at 4°C.

### Sterile filtration

Two sterile filtration steps were performed using a closed system of two steel vessels and filter unit containing 0.22µm PVDF membranes (Milipak60, Durapore). All sterile filtration steps are performed at 4°C.

### Freeze-drying

Freeze-drying was performed according to standard procedures.

### Reconstitution of the lyophilisates

Reconstituted lyophilisates must be protected from light and temperature.

Lyophilisates can be reconstituted with pure water or with a 0.5 mM NaHCO₃ solution.

### Analysis of the manufacturing process (scale of 3I)

Most important analytical results are summarized in the following figures:
- DOTAP and CPT content
- DOTAP and CPT impurity content (area% in the HPLC chromatogram)
- CPT lactone and pH
- Particle number >1, >10 and >25µm (number per ml)

### Comparison manufacturing at low/high temperature

Performing the manufacturing process at 25°C instead of 4°C leads to increased conversion of CPT carboxylate into its lactone form. Productions runs failed due to clogged extrusion membranes. Measurment of particle revealed that especially the numbers of particles of size >10µm and >25µm were dramatically increased. Microscopic analysis indicated CPT lactone crystals and determination of CPT lactone (by HPLC) confirmed lactone content higher than 10% which usually precipitates out from the formulation.
Working at low temperature minimizes CPT lactone formation during manufacturing.

### Literature

1. Wall ME, Wani MC. Camptothecin and taxol: from discovery to clinic. J Ethnopharmacol 1996;51(1-3):239-53; discussion 253-4.
2. Muggia FM, Creaven PJ, Hansen HH, Cohen MH, Selawry OS. Phase I clinical trial of weekly and daily treatment with camptothecin (NSC-100880): correlation with preclinical studies. Cancer Chemother Rep 1972;56(4):515-21.
3. Moertel CG, Schutt AJ, Reitemeier RJ, Hahn RG. Phase II study of camptothecin (NSC-100880) in the treatment of advanced gastrointestinal cancer. Cancer Chemother Rep 1972;56(1):95-101.
4. Hsiang YH, Liu LF. Identification of mammalian DNA topoisomerase I as an intracellular target of the anticancer drug camptothecin. Cancer Res 1988;48(7):1722-6.
5. Kehrer DF, Soepenberg O, Loos WJ, Verweij J, Sparreboom A. Modulation of camptothecin analogs in the treatment of cancer: a review. Anticancer Drugs 2001;12(2):89-105.
6. Felgner JH, Kumar R, Sridhar CN, Wheeler CJ, Tsai YJ, Border R, et al. Enhanced gene delivery and mechanism studies with a novel series of cationic lipid formulations. J Biol Chem 1994;269(4):2550-61.
7. Zunino F, Dallavalleb S, Laccabuea D, Berettaa G, Merlinib L, Pratesi G. Current status and perspectives in the development of camptothecins. Curr Pharm Des. 2002;8(27):2505-20

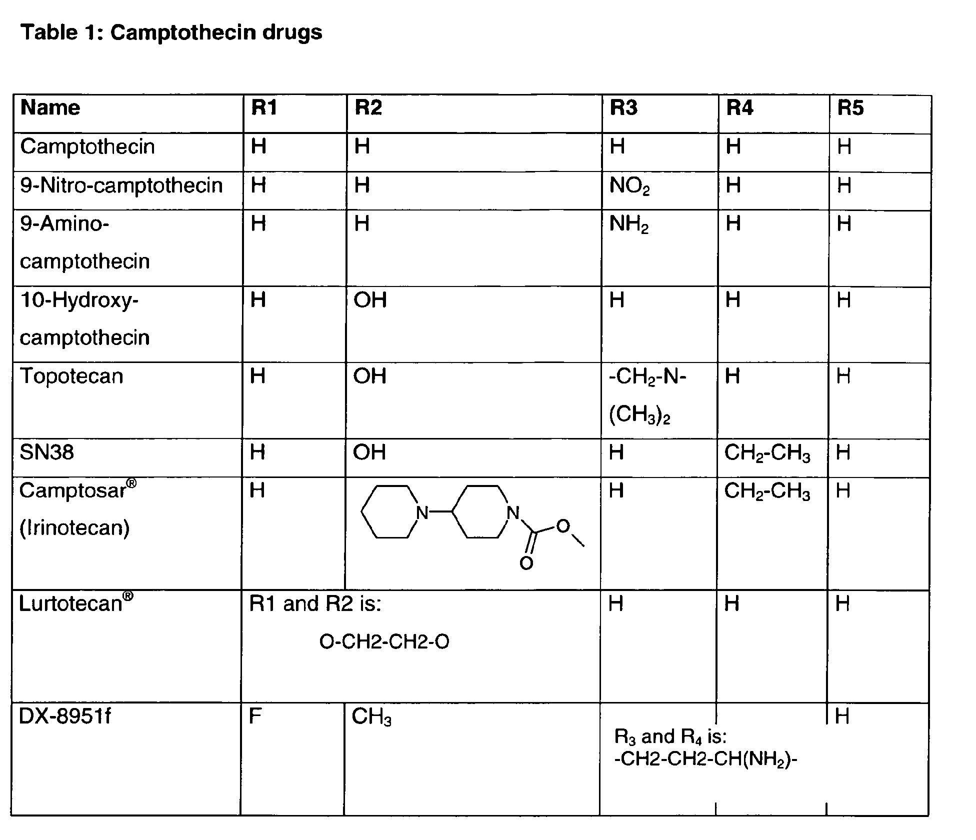

**Table 2:**

| **Preparations manufactured with miscellaneous Tris-buffer concentrations, pH 7.5.** | | | | | | | |
|---|---|---|---|---|---|---|---|
| | | | | **Particles per ml** | | | |
| **Sample** | **Tris** | **Dotap** | **CPT** | **Before lyo** | | **After lyo** | |
| | **[mM]** | **[mM]** | **[mM]** | **>10µm** | **>25µm** | **>10µm** | **>25µm** |
| RM773 | 0 | 10 | 0 | 13 | 3 | 53 | 1 |
| RM774 | 50 | 10 | 0 | 41 | 9 | 96568 | 10620 |
| RM776 | 0 | 9,5 | 0,5 | 54 | 4 | 418 | 10 |
| RM777 | 10 | 9,5 | 0,5 | 21 | 2 | 13129 | 13 |
| RM778 | 50 | 9,5 | 0,5 | 17 | 1 | | |

**Table 3:**

| **Two preparations reconstituted with pure water and with Tris-buffer (10 mM).** | | | | | | | |
|---|---|---|---|---|---|---|---|
| | | **PW reconst. + Vacuum** | | | **Tris-buffer/ PW reconst.+ Vacuum** | | |
| **Sample** | **Tris** | **PAMAS** | | | **PAMAS** | | |
| | **[mM]** | **>1µm** | **>10µm** | **>25µm** | **>1µm** | **>10µm** | **>25µm** |
| RM773 | 0 | 41977 | 7 | 0 | 25585 8 | 2292 | 13 |
| RM776 | 0 | 35718 | 12 | 1 | 96863 | 760 | 3 |

**Table 4:**

| **Results of the PAMAS and DLS measurements** | | | | | | | | | | | | | |
|---|---|---|---|---|---|---|---|---|---|---|---|---|---|
| | | | | | | **Before Lyo** | | | | **After Lyo** | | | |
| | **Dotap** | **CPT** | **Tris** | **Trehalose** | **Reconst.** | **Z**_{**ave**} | **PI** | **PAMAS** | | **Z**_{**ave**} | **PI** | **PAMAS** | |
| **Sample** | **[mM]** | **[mM]** | **[mM]** | **[%]** | | **[nm]** | **[---]** | **>10µm** | **>25µm** | **[nm]** | **[---]** | **>10µm** | **>25µm** |
| RM763 | 23,75 | 1,25 | 10 | 5 | H₂O | 146 | 0,09 | 77 | 21 | 327 | 1,00 | 341390 | 3745 |
| RM764 | 23,75 | 1,25 | 10 | 10 | H₂O | 165 | 0,13 | 48 | 9 | | | 105000 | 1470 |
| RM765 | 19 | 1 | 10 | 5 | H₂O | | | 70 | 56 | | | 290220 | 2870 |
| RM766 | 14,25 | 0,75 | 10 | 5 | H₂O | | | 38 | 9 | | | 173285 | 1680 |
| RM767 | 19 | 1 | 10 | 10 | H₂O | | | 48 | 9 | | | 418705 | 3605 |
| RM768 | 14,25 | 0,75 | 10 | 10 | H₂O | 164 | 0,11 | 48 | 9 | 197 | 0,28 | 105000 | 1470 |
| RM772 | 25 | 0 | 10 | 10 | H₂O | | | 22 | 9 | 158 | 0,15 | | |
| RM772 | 20 | 0 | 10 | 10 | H₂O | | | | | | | | |
| RM772 | 15 | 0 | 10 | 10 | H₂O | | | | | | | | |
| RM772 | 10 | 0 | 10 | 10 | H₂O | | | | | 161 | 0,24 | 35763 | 2348 |
| RM772 | 5 | 0 | 10 | 10 | H₂O | | | | | | | 5118 | 249 |
| RM773 | 10 | 0 | 0 | 10 | H₂O | 158 | 0,12 | 13 | 3 | 188 | 0,39 | 53 | 1 |
| RM774 | 10 | 0 | 50 | 10 | H₂O | | | 41 | 9 | 176 0 | ,36 | 96568 | 10620 |
| RM775 | 10 | 0 | 10 | 5 | H₂O | | | | | 176 | 0,32 | | |
| RM776 | 9,5 | 0,5 | 0 | 10 | H₂O | 163 | 0,11 | 54 | 4 | | | 418 | 10 |
| RM777 | 9,5 | 0,5 | 10 | 10 | H₂O | | | 21 | 2 | 172 | 0,34 | 13129 | 13 |
| RM778 | 9,5 | 0,5 | 50 | 10 | H₂O | | | 17 | 1 | 164 | 0,17 | | |
| RM780 | 15 | 0 | 0 | 10 | H₂O | | | 0 | 0 | 172 | 0,34 | 3 | 0 |
| RM781 | 20 | 0 | 0 | 10 | H₂O | 158 | 0,28 | 0 | 0 | 188 | 0,39 | 3 | 1 |
| RM782 | 25 | 0 | 0 | 10 | H₂O | 151 | 0,31 | 0 | 0 | 175 | 0,41 | 18 | 1 |
| RM783 | 30 | 0 | 0 | 10 | H₂O | 148 | 0,32 | 1 | 0 | 175 | 0,34 | 24 | 1 |
| RM784 | 14,25 | 0,75 | 0 | 10 | H₂O | | | 0 | 0 | 165 | 0,15 | 2 | 0 |
| RM785 | 14,25 | 0,75 | 0 | 10 | H₂O | | | 0 | 0 | | | 8 | 2 |
| RM788 | 30 | 0 | 0 | 10 | H₂O | | | 94 | 14 | | | | |
| RM789 | 30 | 0 | 0 | 10 | H₂O | | | 72 | 0 | | | | |
| RM790 | 40 | 0 | 0 | 10 | H₂O | | | 384 | 252 | | | | |
| RM791 | 23,75 | 1,25 | 0 | 10 | H₂O | | | 0 | 0 | 156 | 0,14 | | |
| RM792 | 28,5 | 1,5 | 0 | 10 | H₂O | | | 12 | 0 | | | | |
| RM793 | 25,97 | 1,75 | 0 | 10 | H₂O | | | 0 | 0 | | | | |
| RM794 | 24,38 | 0,63 | 0 | 10 | H₂O | | | 8 | 4 | | | | |
| RM801 | 23,75 | 1,25 | 0 | 10 | H₂O | 168 | 0,18 | | | 141 | 0,27 | 12 | 1 |
| RM805 | 23,75 | 1,25 | 0 | 9 | H₂O | 165 | 0,21 | 3 | 0 | 151 | 0,41 | 19 | 0 |
| RM806 | 23,75 | 1,25 | 0 | 9 | H₂O | 170 | 0,18 | 2 | 0 | 158 | 0,40 | 8 | 1 |
| RM807 | 28,5 | 1,5 | 0 | 9 | H₂O | 170 | 0,17 | 0 | 0 | 161 | 0,49 | 10 | 0 |
| RM808 | 30 | 0 | 0 | 9 | H₂O | 162 | 0,27 | 3 | 0 | 152 | 0,40 | 15 | 0 |
| RM815 | 28,5 | 1,5 | 0 | 8,5 | H₂O | 168 | 0,16 | 10 | 0 | 169 | 0,43 | 30 | 0 |
| RM816 | 29,25 | 0,75 | 0 | 8,5 | H₂O | 161 | 0,19 | 10 | 0 | 203 | 0,70 | 100 | 0 |
| RM817 | 14,25 | 0,75 | 0 | 8,5 | H₂O | 165 | 0,17 | 55 | 5 | 169 | 0,38 | 35 | 0 |
| RM818 | 30 | 0 | 0 | 8,5 | H₂O | 154 | 0,26 | 10 | 0 | 183 | 0,65 | 60 | 0 |
| RM840 | 23,75 | 1,25 | 0 | 8,5 | H₂O | 175 | 0,18 | 80 | 0 | 181 | 0,39 | 20 | 0 |

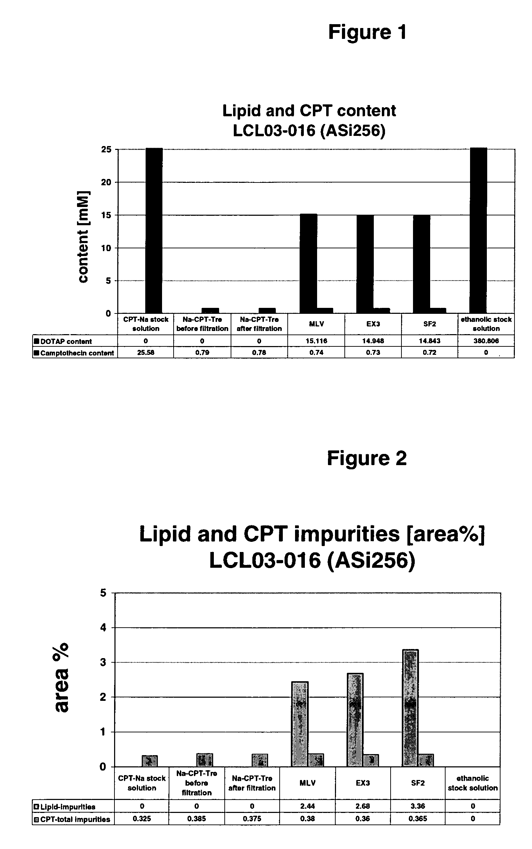

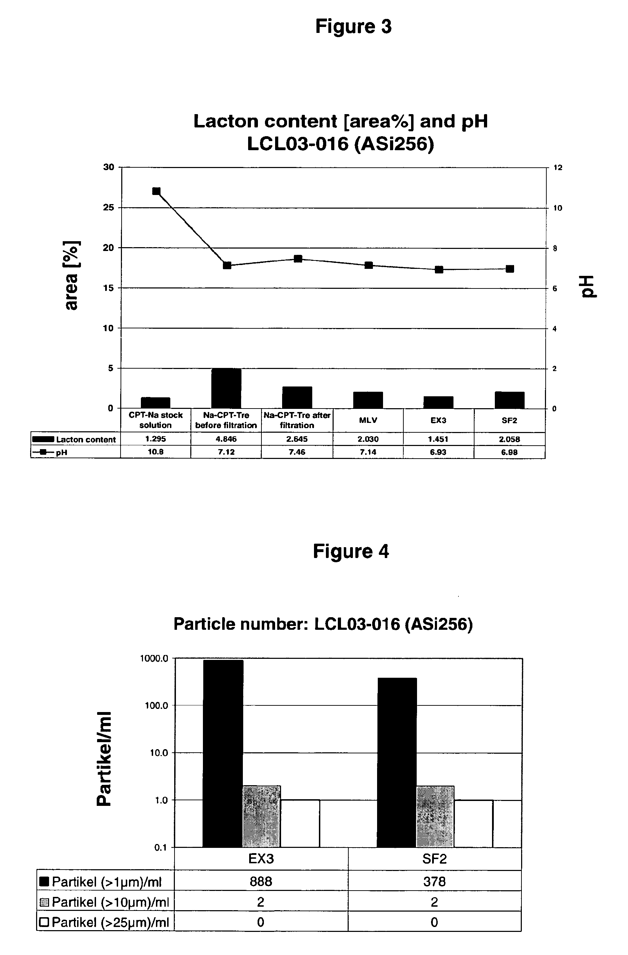

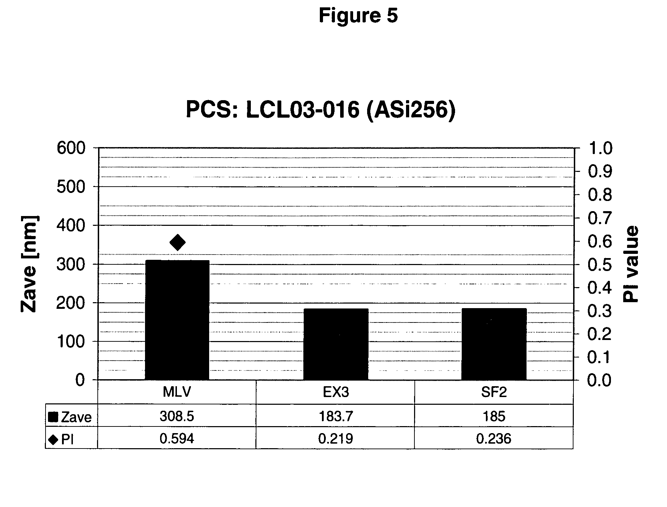

## Claims

1. Method of producing a cationic liposomal preparation comprising a camptothecin drug in its carboxylate form, comprising the steps of
f) providing cationic liposomes in an aqueous medium comprising the components
i) at least one cationic lipid and optionally at least one amphiphile,
ii) a camptothecin drug in its carboxylate form and
iii) a cryoprotectant,
g) optionally homogenizing the liposomes of step a) at least once,
h) optionally sterile filtrating the liposomes of step a) or b),
i) dehydrating the liposomes of step a) b) or c) and
j) reconstituting the dehydrated liposomes of step d) in an aqueous medium,
wherein said aqueous medium of step a) and/or of step e) comprises a pH active agent in a concentration of 0 mM to about 10 mM and has a pH between about 5 and about 9, preferably between about 6 and about 8.

2. The method of claim 1, wherein said cationic lipid is in an amount of at least about 30 mol% based on the total lipids of the cationic liposomes.

3. The method of claim 1 or 2, wherein the positively charged group of said cationic lipid is a a tertiary amino or quaternary ammonium group such as N-[1-(2,3-diacyloxy)propyl]-N,N-dimethylamine or N-[1-(2,3-diacyloxy)propyl]-N,N,N-trimethyl ammonium, preferably N-[1-(2,3-dioleoyloxy)propyl]-N,N,N-trimethyl ammonium (DOTAP) or N-[1-(2,3-dioleoyloxy)propyl]-N,N,N-trimethyl ammonium (DOTAP).

4. The method of any one of the claims 1 to 3, wherein said amphiphile is in an amount of up to about 70 mol% based on the total lipids of the cationic liposomes.

5. The method of any one of the claims 1 to 4, wherein said amphiphile is non-cationic and preferably selected from sterols such as cholesterol, from phospholipids, lysolipids, lysophospholipids, sphingolipids or pegylated lipids and combinations thereof, preferably diacylphosphatidylcholine.

6. The method of any one of the claims 1 to 5, wherein said camptothecin carboxylate drug is in an amount of at least about 0.1 mol% to up to about 100 mol%, preferably less than about 50 mol% with respect to the total lipid.

7. The method of any one of the claims 1 to 6, wherein said pH active agent is selected from Tris, Hepes, Bis, phosphate, carbonate or amino acids, optionally together with a base or an acid such as NaOH or HCl.

8. The method of any one of the claims 1 to 7, wherein a stabilizing agent is present during at least one of the steps a) to e), wherein said which stabilizing agent is preferably an antioxidant and more preferably selected from alpha-tocopherol or vitamin C.

9. The method of any one of the claims 1 to 8, wherein at least one of the steps, preferably all of the steps a) to e) are performed under avoidance of light.

10. A cationic liposomal preparation comprising a camptothecin drug in its carboxylate form and a pH active agent of up to about 10 mM in an aqueous medium, wherein said medium has a pH between about 5 and about 9, preferably between about 6 and about 8.

11. A cationic liposomal preparation obtainable by a process of any one of the claims 1 to 9.

12. A pharmaceutical composition comprising a liposomal preparation of any one of the claims 10 or 11, optionally together with a pharmaceutically acceptable carrier, diluent and/or adjuvant.
